# EUROPEAN PATENT APPLICATION

(11) **EP 4 674 477 A1**
(43) Date of publication of application: **07.01.2026**
(21) Application number: 24763989.1
(22) Date of filing: 28.02.2024
(51) Int. Cl.: A61N 5/10

(54) **DETECTION DEVICE, TRANSPORTABLE TREATMENT TABLE, AND TREATMENT SYSTEM**

(30) Priority: 28.02.2023 JP 2023030495
(71) Applicant: B dot Medical Inc., Tokyo 134-0003 (JP)
(72) Inventor: MINOHARA, Shinichi, Tokyo 134-0003 (JP); KUBOTA, Yoshiki, Tokyo 134-0003 (JP)
(74) Representative: EIP
(86) International application number: PCT/JP2024/007359
(87) International publication number: WO 2024/181511

(57) **Abstract**

To improve safety or convenience of treatment using a detection device, a detection device 5 includes a detection unit 51 that is placed on a treatment table 1 including a placement portion 2 and is configured to detect a surface position and/or a body motion displacement of a living body placed on the placement portion 2, a battery 6 capable of supplying power to the detection unit 51, in a state of being disconnected from an external power supply facility; a communication unit 52 configured to wirelessly transmit, to an external device, data corresponding to a signal output from the detection unit 51; and a connection unit 53 configured to connect the detection unit 51 to the treatment table 1, the detection unit 51 being connected to be movable in accordance with a movement of the placement portion 2 that is movable on the treatment table 1.

## Description

### Technical Field

The present disclosure relates to a technique for monitoring a surface position and/or a body motion displacement of a living body.

### Background Art

Various known techniques for monitoring a surface position and/or body motion displacement of a living body have been proposed (see Patent Documents 1 to 4 and Non-Patent Document 1). In addition, a treatment table has been proposed in which a top plate for placing a patient thereon is detachable (see Patent Document 5).

### Citation List

### Patent Document

Patent Document 1: Patent No. 3326597
Patent Document 2: Patent No. 3643573
Patent Document 3: Patent No. 5373285
Patent Document 4: JP 2018-192253 A
Patent Document 5: Patent No. 5795903

### Non-Patent Document

Non-Patent Document 1: K. Shioiri, and nine others, "Development of an easy-to-use respiration sensor with fast response for respiratory gated radiation therapy", 56th Annual Conference of the Particle Therapy Co-operative Group, Poster Presentation No. 263, 2017, May, < URL: https://www.czech-in.org/cmgateway/ptcog17/index.html? module=searchableprogramme & personid=anonymous&key=0b043bad6d30b7641c8df4bb4ba50591f39d2bfe #! abstractdetails/0044702>

### Summary of Invention

### Technical Problem

Various known detection devices for monitoring a surface position and/or body motion displacement of a living body have been proposed. However, since all of these detection devices require cables for power supply or communication to be routed, there is a problem in terms of safety or convenience of treatment.

In view of the above problem, an object of the present disclosure is to improve safety or convenience of treatment using a detection device.

### Solution to Problem

An example of the present disclosure is a detection device comprising: a detection unit that is installed on a treatment table including a placement portion on which a living body is placed, and configured to detect a surface position and/or a body motion displacement of the living body placed on the placement portion; a battery that is movable together with the treatment table, and capable of supplying power to the detection unit, in a state of being disconnected from an external power supply facility; a communication unit configured to wirelessly transmit, to an external device, data corresponding to a signal output from the detection unit; and a connection unit configured to connect the detection unit to the treatment table, the detection unit being connected to be movable in accordance with a movement of the placement portion that is movable on the treatment table.

An example of the present disclosure is a transportable treatment table that is transportable to another location while a living body is placed thereon, the transportable treatment table comprising: a placement portion on which the living body is placed; a detection unit configured to detect a surface position and/or a body motion displacement of the living body placed on the placement portion; a battery that is movable together with the transportable treatment table, and capable of supplying power to the detection unit, in a state of being disconnected from an external power supply facility; a communication unit configured to wirelessly transmit, to an external device, data corresponding to a signal output from the detection unit; a connection unit configured to connect the detection unit to the transportable treatment table, the detection unit being connected to be movable in accordance with a movement of the placement portion that is movable on the transportable treatment table; and a transport unit making the transportable treatment table to be movable.

An example of the present disclosure is a treatment system comprising: a transportable treatment table that is transportable to another location wile a living body is placed thereon; an irradiation device configured to perform beam irradiation on the living body for treatment; and a signal control device configured to control the irradiation device in accordance with data input, wherein the transportable treatment table includes: a placement portion on which a living body is placed; a detection unit configured to detect a surface position and/or a body motion displacement of the living body placed on the placement portion; a battery that is movable together with the transportable treatment table, and capable of supplying power to the detection unit, in a state of being disconnected from an external power supply facility; a communication unit configured to wirelessly transmit, to an external device, data corresponding to a signal output from the detection unit; a connection unit configured to connect the detection unit to the transportable treatment table, the detection unit being connected to be movable in accordance with a movement of the placement portion that is movable on the transportable treatment table; and a transport unit making the transportable treatment table to be movable, and the signal control device includes: a data reception unit configured to receive the data wirelessly transmitted from the communication unit; and a control signal output unit configured to output a signal for enabling control of the beam irradiation by the irradiation device, based on the data received.

An example of the present disclosure is a detection device comprising: a detection unit that is installed on a treatment table including a placement portion on which a living body is placed, and configured to detect a surface position and/or a body motion displacement of the living body placed on the placement portion; and a reflector installed on the living body, wherein the detection unit includes a light source and a light position detection sensor, and an optical path is configured so that incident light emitted from the light source to the living body is reflected by the reflector and reflected light from the reflector is input to the light position detection sensor.

The present disclosure can be recognized as an information processing apparatus, a system, a method executed by a computer, or a program executed by a computer. In addition, the present disclosure can be recognized as a recording medium from which such a program can be read by a computer, other devices, machines, or the like. Here, the recording medium readable by a computer or the like refers to a recording medium in which information such as data and programs can be stored by electrical, magnetic, optical, mechanical, or chemical action, to be readable by a computer or the like.

### Advantageous Effects of Invention

According to the present disclosure, it is possible to improve the safety or convenience of treatment using a detection device.

### Brief Description of Drawings

FIG. 1 is a diagram illustrating an example of a system configuration according to an embodiment.
FIG. 2 is a diagram illustrating an example of a configuration in an external appearance of a transportable treatment table according to an embodiment.
FIG. 3 is a diagram (1) schematically illustrating an optical path formed in a detection device according to an embodiment.
FIG. 4 is a diagram (2) schematically illustrating the optical path formed in the detection device according to an embodiment.
FIG. 5 is a diagram (3) schematically illustrating the optical path formed in the detection device according to an embodiment.
FIG. 6 is a diagram (4) schematically illustrating the optical path formed in the detection device according to an embodiment.
FIG. 7 is a diagram (5) schematically illustrating the optical path formed in the detection device according to an embodiment.
FIG. 8 is a diagram schematically illustrating a configuration of a signal control port according to an embodiment.
FIG. 9 is a diagram illustrating an example of use of the detection device in treatment for a respiratory moving organ according to an embodiment.
FIG. 10 is a diagram illustrating an example of a respiratory waveform received by the signal control port and corresponding gate signal generation and beam irradiation control, according to an embodiment.
FIG. 11 is a diagram illustrating an example of a flow of treatment using the transportable treatment table according to an embodiment.
FIG. 12 is a diagram illustrating an example of information displayed on a display operation unit (display unit) according to an embodiment.

### Description of Embodiments

In a treatment involving radiation beam (photon beam or particle beam) irradiation, in order to accurately irradiate a respiratory moving organ with the beam, respiration synchronous irradiation is performed in which the position of the organ is recognized and the irradiation is performed in accordance with the movement. A method for synchronization for the respiration synchronous irradiation includes an external synchronization method in which the irradiation is performed with movement of a tumor in a body indirectly monitored based on a movement of a living body surface, and an internal synchronization method in which the irradiation is performed with the movement in the body directly monitored through X-ray exposure. The former is a treatment method with which the movement of the tumor and the movement of the living body surface are correlated in advance so as not to involve the X-ray exposure. In the treatment involving beam irradiation, the irradiation is also performed on children, elderly people with dementia, and patients who exhibit involuntary movement. This means that preferably, the body motion near the irradiation region is monitored even for the irradiation site that only needs to be fixed with a fixing tool in general cases, and to interrupt the irradiation in response to a change exceeding a clinically acceptable change.

In the field of diagnostic imaging (such as CT, MRI, and PET) of a trunk portion, the imaging (4D imaging) is preferably performed at an appropriate respiratory timing (such as expiration and inspiration) so as not to obtain an image with artifacts, and at high speed under free respiration to synchronize the respiratory phase information with the imaging timing. Therefore, in image diagnosis, the position on a living body surface is measured, and image processing is performed using information corresponding to the respiratory phase.

Here, in order to measure the movement of the living body surface of a patient, a method has been employed in which the living body surface of the patient is directly measured or in which the living body surface of the patient is indirectly measured with an object serving as a measurement point installed, regardless of the content of medical care. For the method of in which the displacement on the living body surface is directly measured, a strain gauge, a laser displacement gauge, or a laser rangefinder may be used. When used, the strain gauge is attached to the living body surface under a fixing tool for fixing a patient to a treatment table, since it is difficult to perform accurate measurement on a surface of the fixing tool. However, it is difficult to adjust the sensitivity after the fixing tool is attached, and the adjustment is performed for each patient meaning that the setting therefor takes time.

For example, when used, the laser displacement gauge or the laser rangefinder is installed in a direction substantially perpendicular to a living body surface in the vicinity of a patient. Thus, there is a possibility of interference with an imaging and irradiation device and interference with an imaging visual field and an irradiation visual field thereof, and thus, the detection device installation position is adjusted for each patient.

For example, a detection device has been proposed which acquires a living body surface image of a patient or an image of a marker installed on the living body surface using an image from a two-dimensional or three-dimensional camera installed on a ceiling or a wall surface, to measure displacement on the living body surface. However, with such a scheme, since the measurement is performed by a stereo camera including a plurality of CCD cameras installed, in the case of general radiotherapy, the measurement may fail to be performed with the field of view of the camera blocked due to the positional relationship between an irradiation port rotationally moving and the patient. In addition, since a video image is transmitted and subjected to image processing for the position measurement, it takes time and the response time is delayed. In actual medical care, the delay time of position detection may lead to compromised accuracy of image reconstruction, resulting in a failure to perform accurate image diagnosis and evaluation, or a shift in the irradiation position during treatment.

In the above-described known living body surface measurement using the strain gauge, the laser displacement gauge, the laser rangefinder, or the camera installed on the treatment table, the detection device is installed around the patient, and in any case, cables are routed for supplying power to a measurement system device and for transmitting a signal indicating the measurement position. Therefore, the cable is routed around the patient, and this affects the workability of the medical staff during medical care and the safety of the patient.

Specifically, the treatment table of the patient or a treatment table mounting portion moves so that the imaging position and the irradiation position of the treatment table of the patient can be adjusted. Thus, the routed cable or cord may be unexpectedly caught by the treatment table driven. This means that a cumbersome process of detaching and attaching the cable for each use is required, and that the cable may be damaged. When the cables are laid on the floor of a clinical room, the patient and medical staff may stumble, meaning that there is a problem in workability and safety. In addition, when the length of the cable is limited for safety, the setting may not be possible depending on the medical site of interest of the patient.

When the camera is installed on the ceiling, a wide space may be secured around the patient. However, since a video signal is transmitted, it takes about 100 ms for image acquisition and tracking by software image processing. The detection device using a camera image is plagued by a delay in response time. In addition, the field of view of the camera may be blocked by the operation of the treatment table and irradiation device.

In addition, the known detection device transmits a detection signal by wired transmission. This is because wireless transmission involves unstable and slow communication speed. For example, a wireless LAN (4G) used for the signal transmission involves the delay time of 20 ms or longer. When the signal transmission method is changed from wired to wireless, a delay occurs in addition to the known delay in response time. While the medical treatment is preferably interrupted immediately in response to an unexpected interruption of the signal transmission, the wireless transmission of the signal transmission has problems in stability and speed, which affects the accuracy of the medical treatment.

In radiotherapy, multiportal irradiation (irradiation performed by changing an irradiation angle in order to reduce exposure to normal tissues) that delivers irradiation from a plurality of directions in a single treatment session may be performed. In this case, a placement portion on which the patient is placed may be largely rotated during a single treatment session. In the case of the respiration synchronous irradiation, since the period during which irradiation is possible is limited compared with the irradiation on a stationary organ, the total beam ON/OFF control time may be 5 to 20 times the normal irradiation time (normally within one minute). Therefore, the treatment table may be moved for each portal to perform irradiation (for example, the treatment table may be rotated by 180 degrees to perform irradiation from the opposite direction). Since the detection device is wired, the attachment and detachment work of the cable is required which is time consuming. Thus, the accuracy of posture maintenance of the patient is compromised, resulting in compromised treatment accuracy.

In addition, in order to improve the accuracy of patient positioning for reproducing the same posture of the patient as that at the time of imaging of the treatment plan CT as much as possible for each treatment, facilities that introduce an in-room CT in a treatment room have been increasing in recent years. The patient positioning typically uses an X-ray imaging device to acquire two-dimensional images, and the positioning is performed using stationary landmarks (bone structures for example) within the body. However, in the case of a respiratory moving organ, positioning based only on a bone structure is insufficient. Thus, positioning is performed in consideration of a relative positional relationship between the position of a tumor itself and other surrounding organs. Since most tumors are soft tissues meaning that a normal two-dimensional X-ray image is insufficient, the positioning is performed using a CT image enabling soft tissues to be captured in a three-dimensional image. At this time, the CT is installed at a position relatively distant from the irradiation device for the purpose of shielding to be free of the influence of the secondary radiation from the irradiation device. In this context, when a wired detection device is used, the detection device is attached and detached and reset each time, resulting in low work efficiency.

The embodiments described below solve at least one or more of the problems in the known technique described above. Note that an embodiment that employs the technique according to the present disclosure may solve at least a part of the problems of the known technique described above, and may not necessarily solve all of the problems.

Hereinafter, embodiments of a detection device, a transportable treatment table, and a treatment system according to the present disclosure will be described with reference to the drawings. However, the embodiments described below are merely examples, and the detection device, the transportable treatment table, and the treatment system according to the present disclosure are not limited to the specific configurations described below. When embodying, a specific configuration according to an aspect of the embodiment may be adopted as appropriate, and various improvements and modifications may be made. For example, the treatment table is not limited to a transportable treatment table and may be a transportable treatment table also usable for diagnosis (such as, for example, CT imaging or MRI imaging), or a treatment table fixed in a treatment room or a diagnosis room (such as for example, a CT imaging room or an MRI imaging room).

<First Embodiment>

The present embodiment is described as an embodiment in a case where the technique according to the present disclosure is implemented in a treatment system for treating a patient by performing beam irradiation for the treatment. Still, the technique according to the present disclosure can be widely used to monitor the surface position and/or body motion displacement of a living body, and the application target of the present disclosure is not limited to the example described in the embodiment.

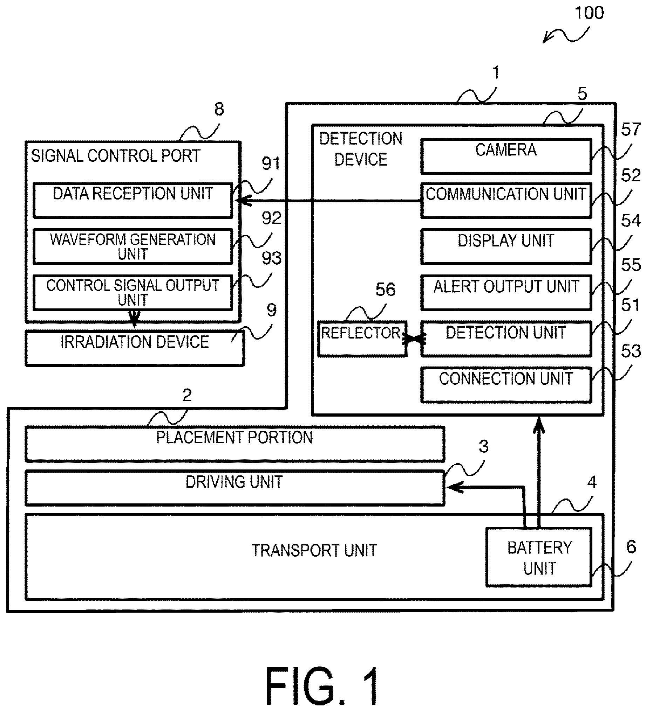

FIG. 1 is a diagram illustrating an example of a system configuration according to the present embodiment. The system according to the present embodiment is a treatment system 100 including a transportable treatment table 1, a signal control port 8, and an irradiation device 9. The transportable treatment table 1 according to the present embodiment is a transportable treatment table 1 that can be transported to another location (for example, from a positioning room to a treatment room) while a living body is placed thereon, and includes a placement portion 2, a driving unit 3, a transport unit 4, and a detection device 5, as well as a battery unit 6 inside the transport unit 4. The detection device 5 includes the signal control port 8 that is provided externally and outputs a signal to the irradiation device 9.

The living body as the target of treatment or procedure is placed on the placement portion 2, which can also be referred to as a bed portion. The placement portion 2 may be provided with a fixing tool or the like for fixing the placed living body. Further, the placement portion may be detachable from the transportable treatment table 1.

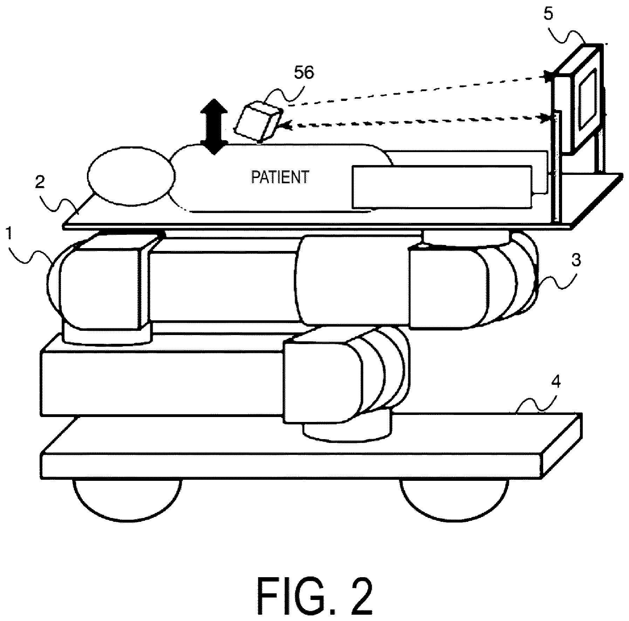

FIG. 2 is a diagram illustrating an example of a configuration in an external appearance of the transportable treatment table 1 according to the present embodiment. As described above, the transportable treatment table 1 includes the placement portion 2, the driving unit 3, the transport unit 4, the detection device 5, and the battery unit 6. A reflector 56 described below is installed on the living body (a patient in the example illustrated in FIG. 2) placed on the placement portion 2.

The driving unit 3 (robot arm unit) is driven upon receiving power supply from the battery unit 6, to translate and/or rotate the placement portion 2. As illustrated in FIG. 2, the driving unit 3 may have a structure of a so-called robot arm, so that the driving unit 3 can cause the placement portion 2 and the living body placed on the placement portion 2 to take various postures.

The transport unit 4 is a mechanism for enabling the transportable treatment table 1 to move (for example, travel) between different locations. The transport unit 4 may be provided with a simple transport assisting mechanism such as wheels or a track that reduces resistance received from a road surface during transport, so that the transportable treatment table 1 can be manually transported, may be capable of assisting the manual transport work by driving wheels or the like upon receiving power supply from the battery unit 6, or may be self-propelling through driving upon receiving power supply from the battery unit 6. The self-propelling transport unit 4 may be capable of moving forward, backward, in a different direction, or the like in accordance with an instruction from the user to allow the movement as desired by the user, or may be capable of automatically moving between predetermined locations by moving along a path programmed in advance.

The detection device 5 (body surface position detection sensor device) is installed on the treatment table and detects the surface position and/or body motion displacement of the living body placed on the placement portion 2. The configuration of the detection device 5 will be described below.

The battery unit 6 is a power supply unit including a storage battery, and is a battery unit capable of supplying power to at least one of the detection device 5, the driving unit 3, and the transport unit 4 in a state of being disconnected from an external power supply facility. In the transportable treatment table 1 according to the present embodiment, the battery unit 6 provided for the transport unit 4 is shared, thereby making it possible to downsize the transportable treatment table 1 and the detection device 5 described below. However, the battery unit may be incorporated in the detection device 5 described below, or may be installed in the vicinity of the placement portion 2, the driving unit 3, or the like of the transportable treatment table 1.

The detection device 5 according to the present embodiment is a detection device 5 for detecting the surface position and/or the body motion displacement of the living body, and includes a detection unit 51, a communication unit 52, a connection unit 53, a display unit 54, an alert output unit 55, the reflector 56, and a camera 57.

The detection unit 51 detects the surface position and/or body motion displacement of the living body placed on the placement portion 2. Here, the specific configuration of the detection unit 51 is not limited as long as the detection unit 51 has a configuration capable of detecting the surface position and/or the body motion displacement of the living body placed on the placement portion 2.

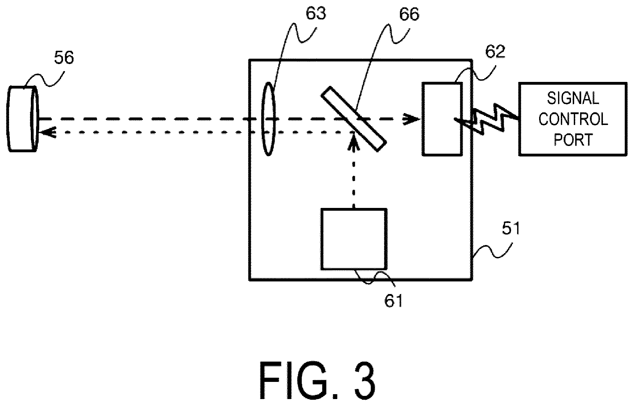

FIG. 3 is a diagram schematically illustrating an optical path formed in the detection device 5 according to the present embodiment. The detection unit 51 in the present embodiment includes a light source 61 and a light position detection sensor 62. The light source 61 emits light on an optical axis (hereinafter, referred to as "the same optical path") that can be regarded as being optically substantially equivalent with that of the light position detection sensor 62, as a result of optical path control by the optical member. The light position detection sensor 62 receives light reflected by the reflector 56 installed (attached) on the living body surface of the patient. That is, the detection unit 51 has an optical path configured so that incident light emitted from the light source 61 to the living body is reflected by the reflector 56, and the reflected light by the reflector 56 is input to the light position detection sensor 62. The light position detection sensor 62 outputs an electric signal corresponding to the position of the light. The electric signal is transmitted to the signal control port 8 (data reception unit 91). Power is supplied to the light position detection sensor 62 and the light source 61 by the battery unit 6, and the signals are transmitted to the signal control port 8 wirelessly.

The detection unit 51 may further include one or a plurality of optical members (such as, for example, a lens 63, a reflection mirror 64, a polarization splitter 65, and a half mirror 66) that form the optical path of the optical system by controlling a trajectory of incident light and/or reflected light. Here, various modes can be adopted for the optical path arranged using the optical members. Such modes will be described below as variations. For example, the detection device 5 illustrated in FIG. 3 is provided with the lens 63 for controlling the incident light and the reflected light, and a half mirror 66 for substantially aligning the optical axis of the incident light and the optical axis of the reflected light in a section between the reflector 56 and the optical member and separating the optical axes in a section between the optical member and the light source 61 or the light position detection sensor 62.

The light position detection sensor 62 is a two-dimensional surface sensor, and an electrical output thereof corresponds to position coordinates (x,y) of the spot light on the sensor surface. For the light position detection sensor 62, for example, a semiconductor position detection element (Position Sensive Detector that is hereinafter referred to as "PSD") may be used. The PSD can directly output voltage signals of two channels x and y in proportion to the position coordinates (x,y) of the spot light without involving software processing. Since no image processing or the like using software is required, a delay in response is small. By utilizing this principle, with the reflector 56 installed on the living body surface of the patient, the spot position on the sensor surface of the reflected light that is displaced in accordance with the variation of the living body surface can be output as a voltage continuously and with a small delay time in proportion to the position coordinates. When spot light from the reflector 56 is incident on the PSD, and the generated photocurrent reaches a resistive layer, charges are extracted in inverse proportion to the distance to each output electrode disposed at the opposite position. The two-dimensional PSD has two sets of output electrodes arranged orthogonally, and can detect the position of the spot light as X and Y coordinates. Thus, the positional displacement of the living body surface is detected two-dimensionally.

The position coordinates (x,y) detected by the light position detection sensor 62 are continuously transmitted as an electric signal to the signal control port 8 wirelessly. The signal control port 8 continuously detects the body motion displacement of the patient as a displacement of a two-dimensional position based on the received signal. When x and y of the position coordinates (x,y) are respectively defined as displacements in the horizontal direction and the vertical direction, the temporal displacements of the position coordinates (x,y) correspond to displacements in the left-right direction and the up-down direction of the living body surface of the patient. In this manner, the treatment system 100 according to the present embodiment continuously monitors the body motion of the patient at the site where the reflector 56 is placed.

The up and down movement of the living body surface of the patient due to respiration is represented by y or √(x² + y²), which can be used as a respiratory waveform. The acquired electric signal is normalized and converted into an external output signal based on an output condition determined in advance for the diagnostic device and the irradiation device. As a result, an irradiation request signal (for example, a gate signal) is transmitted to the diagnostic device or the irradiation device based on a synchronization signal to perform irradiation control so that irradiation for medical care can be performed at an appropriate timing.

In the present embodiment, the semiconductor detector such as a PSD is used for the light position detection sensor 62, so that the position is directly detected as a continuous electric signal. Therefore, according to the present embodiment, the response time from the actual body motion to the data transmission is reduced as compared with the case of adopting the known position detection using CCD camera and image processing, and improvement is achieved in terms of the delay from the body motion detection to the beam irradiation control.

Further, with the PSD used for the light position detection sensor 62 and an infrared LED used for the light source 61, it is possible to suppress power consumption compared with a case where a CCD camera or the like involving large power consumption is used for body surface position detection. Thus, there is an advantage in terms of battery driving.

The communication unit 52 wirelessly transmits data corresponding to the signal output from the detection unit 51 to an external device such as the signal control port 8. In the present embodiment, high-speed wireless communication such as the fifth generation mobile communication system (hereinafter, "5G") is used for a transmission method, thereby reducing a delay due to data transfer (theoretically, about 1 ms). Therefore, according to the present embodiment, the delay time due to data transmission is reduced as compared with the case of using a known wireless communication technique such as a wireless local area network (LAN) or a fourth generation mobile communication system (hereinafter, referred to as "4G"), and the improvement is achieved in terms of the delay from the body motion detection to the beam irradiation control. The 5G communication to be used may be a 5G provided by a communication company, or may be a local 5G in which a system can be constructed limitedly within a site. By using 5G for the communication, even with a wireless transmission system, it is possible to realize suppression of a delay time and stabilization equivalent to wired transmission.

The connection unit 53 is a connection unit 53 that connects the detection unit 51 to the treatment table. The detection unit 51 is connected to be movable in accordance with the movement of the placement portion 2 that is movable in the treatment table. In the present embodiment, the connection unit 53 connects the detection unit 51 to the treatment table so that the relative positional relationship between the living body placed on the placement portion 2 and the detection unit 51 is maintained by fixing the detection unit 51 to the placement portion 2.

The display unit 54 (display) displays the surface position and/or the body motion displacement detected by the detection unit 51. The display unit 54 may be connected to an input device such as a keyboard, a mouse, or a touch panel so as to be capable of receiving an input from a user.

The alert output unit 55 outputs an alert when a preset condition is satisfied by the surface position and/or the body motion displacement detected by the detection unit 51.

The reflector 56 is installed on the living body (for example, attached to the living body surface) to reflect light emitted from the light source 61 of the detection unit 51 toward the living body, to make the light return to the detection unit 51. While a member that can be adopted as the reflector 56 is not limited, in the present embodiment, the retroreflector 56 that reflects light in a direction toward the light source 61 on which the light is incident regardless of a direction in which the light is incident, is used as the reflector 56. The retroreflector 56 may be, for example, a corner cube, and is a device in which three flat mirrors are combined at right angles to each other to form a solid angle apex form. The incident light is reflected a plurality of times by the flat surfaces, and thus the incident light is reflected in the direction toward the light source 61 regardless of the direction of the incident light.

By arranging the light position detection sensor 62 and the light source 61 on the optical axes (the same optical path) that can be regarded as optically substantially equivalent and installing the retroreflector 56 on the living body surface, the reflected light is reflected in the direction toward the light source 61, that is, to the surface of the light position detection sensor 62 on the same optical path, as long as the reflection surface of the retroreflector 56 is set substantially correctly. On the other hand, a reflector (such as a flat mirror) without retroreflection properties has a reflection angle with respect to incident light. Therefore, when a reflector without retroreflection properties is used, it is preferable to finely adjust the reflection surface and the surface of the light position detection sensor 62 so that they accurately face each other, in order to allow the reflected light from the reflector to enter the surface of the light position detection sensor 62 as spot light. In the present embodiment, by using the retroreflector, the orientation adjustment of the reflection surface of the reflector is not required, and the installation on the living body surface of the patient can be easily performed in a short period of time. In addition, when a corner cube having a size of about 1 cm and a weight of several grams (for example, 10 g) or less is used as the retroreflector 56, discomfort and burden on a patient can be reduced. Furthermore, when the reflector 56 used is not made of metal, it is possible to reduce image artifacts in X-ray imaging or CT imaging.

In general, the movement of the living body surface, particularly respiratory movement, is not a simple reciprocating motion but a three-dimensionally complicated motion (AP: up-down direction, LR: left-right direction, SI: body axis direction). In addition, the living body surface has complicated irregularities. Therefore, with the known reflector 56 used without retroreflection properties, it is difficult to set the reflector 56, installed on the living body surface, to capture the trajectory of the respiratory movement for one cycle. Further, since the reflector 56 without retroreflection properties has a wider range for receiving the movement of the spot light than the retroreflector 56, it is necessary to shorten the focal length or widen the detection region of the detection device in order to maintain the resolution of the position detection. If the detection device is placed near the living body in order to shorten the focal length, there is a risk that interference with the diagnostic modality may occur, the irradiation field may be obstructed, or the detection device itself may malfunction or be damaged by the treatment beam. In addition, in a case where a detection device having a large detection region is used, the size of the detection device main body increases, and the size of the power receiving device increases for the driving of the detection device. Thus, the occupation of the space around the living body and the routing of the cord become factors obstructing the movement line of the operator and the movement of the treatment table.

A light source such as an LED may be installed on the living body surface instead of a method of installing the reflector 56 on the living body surface, but this requires a power supply cable to be connected to the LED laid on the living body surface. Further, fine adjustment of the orientation is also performed so that the LED surface and the sensor surface of the detection device optically face each other.

In view of the above, in the present embodiment, the retroreflector 56 is used as the reflector 56. Since the reflector 56 has the retroreflection properties, even when the reflector 56 is roughly installed on the living body surface, the reflected light is reflected in the original direction regardless of the incident direction of the incident light. For example, the surface position monitoring can be easily set by installing a simple camera 57 for collimation in the detection device 5 and confirming that the trajectory of the reflector 56 for one respiration cycle falls within a certain angle of view of the camera 57 (the region of the effective field of view of the light position detection sensor 62). Further, by using the retroreflector 56, the reflected light returns to the same position regardless of the angle of incidence of the incident light, and thus, it suffices if the detection region corresponds to the displacement, whereby the detection device 5 can be easily downsized.

The camera 57 is a collimation camera 57 capable of capturing an image in substantially the same direction as the direction in which the reflected light from the reflector 56 is obtained. By providing the collimation camera 57, it becomes easy to set the detection device 5 so that the detection region of the detection device 5 appropriately captures the reflector 56 on the living body surface. The user who performs the setting only needs to adjust the orientation (height, azimuth angle, elevation angle) of the main body of the detection device 5 so that the reflector 56 and the reflector 56 on the monitor of the collimation camera 57 are displayed substantially near the center of the screen. At this time, if a monitor is provided on the back surface of the main body of the detection device 5 and the collimation camera image is displayed, the user operation is facilitated. The monitor may also display the signal (position coordinates (x,y)) of the light position detection sensor 62, the wireless communication state, the remaining battery level, and the like.

A camera that can be adopted as the collimation camera 57 is not limited, but power consumption can be suppressed by using, for example, a CMOS camera. Since the detection device 5 according to the present embodiment can be driven by a battery in a cordless manner, the CMOS camera features relatively small low consumption compared with other camera sensors is advantageous in order to guarantee a longer operation time. In addition, since the collimation camera 57 is mainly or only aimed at collimation during setting of the detection device 5 and the priority of transmission of the video image is low, transmission of the video image to an external device such as the signal control port 8 may not be performed. When the wireless transmission of the video image is omitted, the target of the wireless transmission is only the data (electric signal) of the position detection. Thus, the high-speed wireless transmission can be maintained, as in the above-described embodiment. That is, with the combination of the PSD and the CMOS camera proposed in the present embodiment, it is possible to realize the same usability as that in the case of using a high-quality CCD camera.

The irradiation device 9 according to the present embodiment performs treatment involving irradiation of a living body with a therapeutic beam in accordance with data input from the signal control port 8. The signal control port 8 includes the data reception unit 91, a waveform generation unit 92, and a control signal output unit 93.

The data reception unit 91 receives data wirelessly transmitted from the communication unit 52.

The waveform generation unit 92 generates a waveform (for example, a normalized respiratory waveform) indicating the surface position and/or the body motion displacement of the living body based on the received data (a signal wirelessly transmitted from the detection device 5 that has detected the displacement of the reflected light position as a voltage displacement).

The control signal output unit 93 outputs a signal enabling the beam irradiation by the irradiation device 9 to be controlled based on the received data. More specifically, in the present embodiment, the control signal output unit 93 outputs a signal (beam request signal/gate signal) for controlling beam irradiation to the irradiation device 9 so that beam irradiation is performed at a timing satisfying a predetermined condition (set level) for determining that the surface position and/or body motion displacement of the living body indicated by the waveform is stable.

The irradiation device 9 irradiates the living body placed on the placement portion 2 with a therapeutic radiation beam (photon beam or particle beam).

Each of the above-described configurations may be controlled by a control unit (computer) including a central processing unit (CPU), a read only memory (ROM), a random access memory (RAM), a storage device, an input device, an output device, a communication unit, and the like (not illustrated). The specific hardware configuration of the control unit can be omitted, replaced, or added as appropriate according to the embodiment. The control unit is not limited to a device including a single housing. The control unit may be realized by a plurality of devices using a so-called cloud or distributed computing technique or the like. The control unit causes the detection device 5 to function as an information processing device including the above-described components by loading a program recorded in a storage device 14 onto the RAM and executing the program using the CPU to control each hardware. In the present embodiment and other embodiments described below, each function of the control unit is executed by the CPU which is a general-purpose processor, but a part or all of these functions may be executed by one or a plurality of dedicated processors.

Hereinafter, other embodiments in a case where the technique according to the present disclosure described above is implemented will be described. In the embodiments described in the present disclosure, the same reference numerals are given to the same configurations, and the description thereof will be omitted. In the embodiments described below, a case where a living body whose surface position and/or body motion displacement is to be detected is a person to be treated (hereinafter, referred to as a "patient") will be described. However, a living body to which the technique according to the present disclosure is applicable is not limited to a person to be treated, and the technique may be applied to a person who may or may not be affected by a disease (for example, for the purpose of an examination or the like) or may be applied to a living body other than a human being.

### <Second Embodiment>

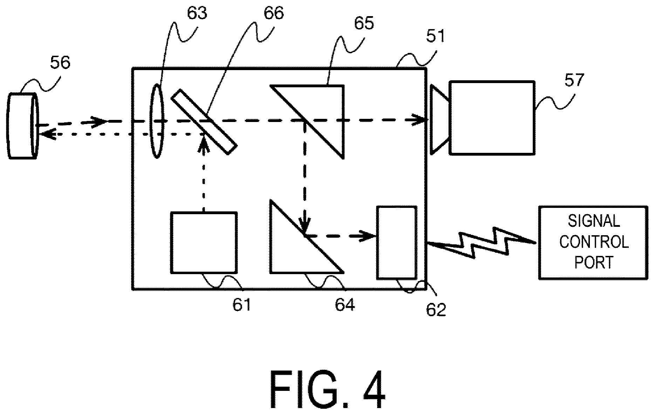

FIG. 4 is a diagram schematically illustrating an optical path formed in the detection device 5 according to the present embodiment. In the present embodiment, an example in which the collimation camera 57 is provided in addition to the configuration in the first embodiment described above will be described. The second embodiment is substantially the same as the first embodiment described above in that the reflector 56 is installed on the living body surface of the patient, light is emitted from the light source 61 in the same direction as the light position detection sensor 62, and the light reflected by the reflector 56 is received by the light position detection sensor 62, that the light position detection sensor 62 outputs an electric signal, corresponding to the position of the light, to the signal control port 8 (data reception unit 91) of the irradiation device 9, that power is supplied to the light position detection sensor 62 and the light source 61 from the battery unit 6, that a signal is transmitted to the signal control port 8 wirelessly, and the like.

By further providing the collimation camera 57, even when the effective field of view that can be monitored by the PSD is narrow, it is possible to easily check the field of view and set the detection device 5 in an appropriate direction. Since the effective area of the PSD sensor is constant, the sensitivity of the PSD to the amount of displacement increases with a decrease in the effective field of view to be monitored (a small change can be detected as a large electric signal). This is advantageous in terms of the performance of the detection device 5. With the example illustrated in FIG. 4, an example is described where the light source 61, the light position detection sensor 62, and the collimation camera 57 are arranged on optical axes (the same optical path) that can be regarded as being optically substantially equivalent.

Optical members such as the lens 63, the reflection mirror 64, the polarization splitter 65, and the half mirror 66 may be used as appropriate to form an optical path of the optical system of the detection device 5. For example, as illustrated in FIG. 4, the collimation camera 57 may be installed at a position to be not coaxial with the position of the light position detection sensor 62 using the polarization splitter 65.

### <Third Embodiment>

When the optical path in the detection device 5 is linear as a whole, the length (depth length) of the main body of the detection device 5 in the patient body axis direction (direction toward the reflector 56) becomes long. In a case where the detection device 5 is installed on the patient's foot side of the placement portion 2 (bed portion/top plate) of the treatment table, the connection unit 53 (frame) supporting the detection device 5 preferably has a small size so as not to interfere with the patient. Still, if the connection unit 53 has a small size and the depth length of the main body of the detection device 5 is long, a structure for fixing the detection device 5 (for example, a structure for supporting at one point) becomes insufficient against vibration during movement of the treatment table or the like. This results in compromised measurement accuracy due to deviation of the optical axis or a failure of the optical device.

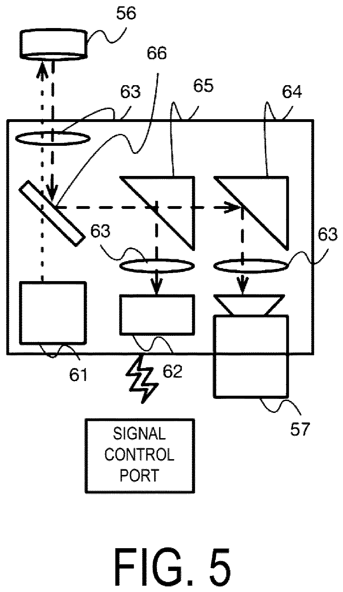

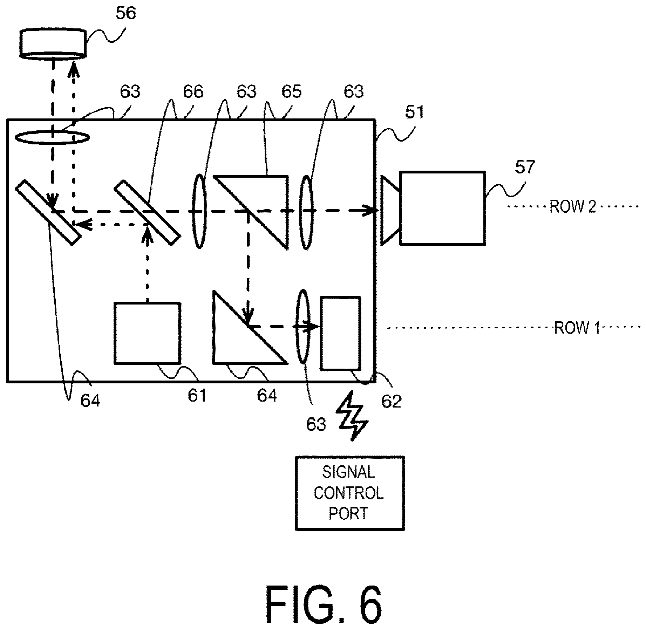

Therefore, in the present embodiment, in order to reduce the depth length of the detection device 5, a configuration is adopted in which the optical path is bent using one or a plurality of optical members. As illustrated in FIG. 5 or 6, by bending the optical path in a vertical plane with respect to the body axis direction of the patient, the depth length of the detection device 5 is shortened to reduce the size of the entire detection device 5. With this configuration, the detection device 5 can be supported in a well-balanced manner even if the connection unit 53 has a small size, and sufficient fixation against vibration can be guaranteed.

Here, at least one optical member among the one or plurality of optical members may be the half mirror 66. The half mirror 66 substantially aligns the optical axis of the incident light and the optical axis of the reflected light in a section between the reflector 56 and the half mirror 66, and separates the optical axes in a section between the half mirror 66 and the light source 61 or the light position detection sensor 62.

At least one optical member of the one or plurality of optical members may be the reflection mirror 64. The reflection mirror 64 is provided in a section between the reflector 56 and the light position detection sensor, thereby controlling the optical axis of the incident light and the optical axis of the reflected light in the section.

At least one optical member of the one or plurality of optical members may be the polarization splitter 65. The polarization splitter 65 separates the input light from the living body side including the reflected light into light that includes at least the reflected light to enter the light position detection sensor 62 and light that includes at least the visible light from the living body side to enter the camera 57.

FIG. 5 is a diagram schematically illustrating an optical path formed in the detection device 5 according to the present embodiment. In the example illustrated in the figure, the optical path is bent in a vertical plane with respect to the body axis direction of the patient using the half mirror 66, the polarization splitter 65, and the reflection mirror 64, and the light source 61, the PSD (light position detection sensor 62), and the collimation camera 57 are substantially aligned, whereby the optical path in the patient body axis direction (direction directed to the reflector 56) is shortened. The light emitted from the light source 61 and passed through the half mirror 66 travels as incident light toward the retroreflective reflector 56 (for example, a corner cube) installed on the living body surface of the patient. The light reflected by the reflector 56 is retroreflected toward the half mirror 66. The reflected light is separated by the half mirror 66, and then further separated by the polarization splitter 65 into light received by the PSD and light reflected by the reflection mirror 64 and received by the collimation image camera 57. Note that an appropriate optical member may be used for the polarization splitter 65, the mirror, and the like depending on the purpose.

FIG. 6 is a diagram schematically illustrating an optical path formed in the detection device 5 according to the present embodiment. In the example illustrated in the figure, the light source 61 and the PSD are installed on a row 1, and the collimation camera 57 is arranged on a row 2 substantially parallel to the row 1, whereby the optical path in the patient body axis direction (the direction toward the reflector 56) is shortened. Light emitted from the light source 61 installed substantially on the row 1 is separated by the half mirror 66 installed on the row 2, and the separated light is further reflected by the reflection mirror 64 installed on the row 2 and travels as incident light toward the retroreflective reflector 56 (for example, a corner cube) installed on the living body surface of the patient. The reflected light from the reflector 56 is retroreflected toward the reflection mirror 64 on the row 2. The reflected light is reflected by the reflection mirror 64, then travels on the row 2, the optical path is separated by the polarization splitter 65, the reflected light is reflected by the reflection mirror 64 installed on the row 1, the reflected light travels on the row 1, and the reflected light is received by the PSD. On the other hand, the reflected light transmitted through the polarization splitter 65 travels on the row 2 and is received by the collimation image camera 57. As described above, an appropriate optical member may be used for the polarization splitter 65, the mirror, or the like depending on the purpose.

The length (depth length) in the patient body axis direction (direction toward the reflector 56) is shortened, while the length in the vertical plane with respect to the patient body axis direction is widened, but the depth length can be made shorter than the known depth length by devising the optical path design as described above. Therefore, the connection unit 53 of the detection device 5 can be easily changed from a structure in which the main body of the detection device 5 is supported at one point to a structure in which the main body of the detection device 5 is supported at both sides of the device (see FIG. 2). Since the known connection unit has a structure that supports the main body of the detection device 5 at one point, there is a possibility that the fixing position to the treatment table is limited, and the main body partially protrudes from the placement portion and may interfere with a surrounding device. On the other hand, by adopting a structure in which the main body of the detection device 5, which is downsized, is supported on both sides, the main body of the detection device 5 does not protrude from the treatment table, and interference with surrounding devices is suppressed. The fixing can be performed not only at a limited place such as the end of the placement portion 2 of the treatment table but also at an arbitrary position of the placement portion 2 with both sides of the placement portion 2 serving as the fulcrum. Therefore, the detection device 5 can be firmly fixed, and the influence of vibration or the like, caused by movement of the treatment table, on the detection device 5 can be suppressed. Further, a mechanism for finely adjusting the angle of the detection device 5 can be attached with the fulcrum being on both sides, and the adjustment is facilitated. In addition, by attaching rails to both sides of the placement portion 2 in the patient body axis direction and providing a mechanism in which the connection unit 53 can move on the rails, the detection device 5 can be installed at an appropriate position closer to a patient with a short height such as a child, and the detection capability for the movement of the living body surface can be maintained for various body positions of various patients. In general, the placement portion 2 has a plurality of holes at equal intervals on both sides in the patient's body axis direction. These holes are for fixing the fixing tool with commercially available bars that are placed in the patient's left-right (LR) direction. It is also possible to use these holes to install the connection unit 53 at an arbitrary position.

### <Fourth Embodiment>

In the case of using the retroreflective reflector 56 such as a corner cube, as long as the light source 61 and the PSD are located on the same optical path, the collimation camera 57 does not necessarily have to be installed on the same optical path. Since the reflector 56 is expected to be installed within a certain distance from the detection unit 51 (for example, within a range of about 1 m from the trunk portion to the feet), the range of the installation angle of the collimation camera 57 for capturing the motion of the reflector 56 falls within a predetermined range. Therefore, if the angle formed by the optical axis of the incident light/reflected light with respect to the reflector 56 and the optical axis of the collimation camera 57 falls within an appropriate predetermined range, the collimation camera 57 may be installed outside the optical axis of the incident light/reflected light.

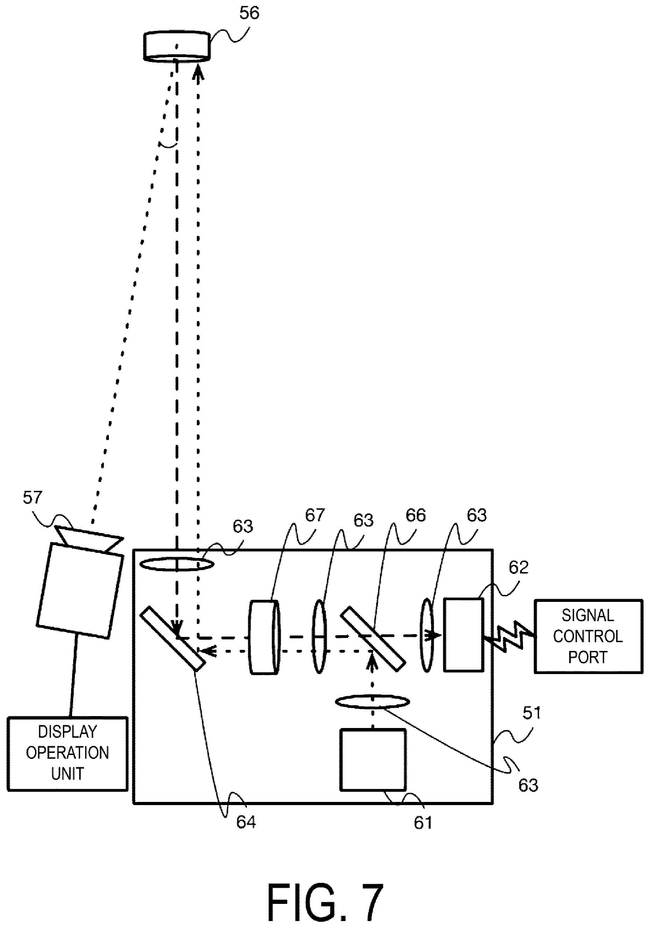

FIG. 7 is a diagram schematically illustrating an optical path formed in the detection device 5 according to the present embodiment. In the example illustrated in FIG. 7, the collimation camera 57 is provided outside the optical axis of the incident light/reflected light, thereby enabling a simple optical path to be formed and the entire optical path to be shortened. As described above, the angle formed by the optical axis of the incident light/reflected light with respect to the reflector 56 and the optical axis of the collimation camera 57 is kept within an appropriate predetermined range in which the collimation camera 57 can capture the movement of the reflector 56. In addition, with a zoom lens 67 installed on the optical axis, it is possible to increase the detection capability even when the amplitude of the living body surface is small. The zoom lens 67 used here may be an electric zoom lens enabling a zoom operation by a motor driven in accordance with a user instruction via a user interface such as a touch panel provided in the detection device 5, or may be a manual zoom lens that is zoomed by a user directly turning the zoom lens.

### <Fifth Embodiment>

In order to perform beam irradiation on an organ moving with respiration highly accurately, the expiratory phase is recognized from a step of acquiring a CT image (hereinafter, referred to as a "treatment plan CT") serving as a reference for creating a treatment plan. For example, when the tumor itself moves due to respiratory movement because the lung, the liver, the pancreas, or the like is the irradiation target, it is a common practice to perform irradiation in accordance with the timing of the expiratory phase in which the displacement of the amplitude is relatively small, and therefore, it is preferable to perform treatment plan CT imaging in the expiratory phase in which the displacement is small. In a respiratory cycle of about 3 to 5 seconds, irradiation is possible in an expiratory phase in which displacement is generally small, and an organ is rapidly displaced before and after the expiratory phase. Therefore, when the synchronization signal is delayed, the irradiation position is displaced, and the irradiation accuracy is compromised. Since the treatment is performed under free breathing, it is preferable to perform the respiration synchronous irradiation in which the treatment beam is irradiated in accordance with the expiratory phase. An example of the respiration synchronous irradiation will be described below.

In the present embodiment, first, as the treatment plan CT, 4D CT or 4D MRT imaging is performed in consideration of time. In the case of 4D imaging, a plurality of 2D or 3D images are acquired while changing the position in an acquisition target range. At the same time, the displacement amount of the living body surface detected by the detection device 5, that is, the respiratory waveform is also acquired, and the acquired image and the expiratory waveform are temporally and spatially rearranged in association with each other. For example, in the case of CT, the bed or the CT itself is moved by self-driven and imaging is performed while changing the position. In the case of MRI, a sagittal cross-section or a body axis cross-section is acquired for each respiratory cycle. For example, the respiratory waveform acquired at this time is used as a reference respiratory waveform in the treatment. Then, the displacement of the tumor in the body on the 4D image is correlated with the reference respiratory waveform before the treatment, and the set level of the respiratory waveform signal for beam irradiation is determined.

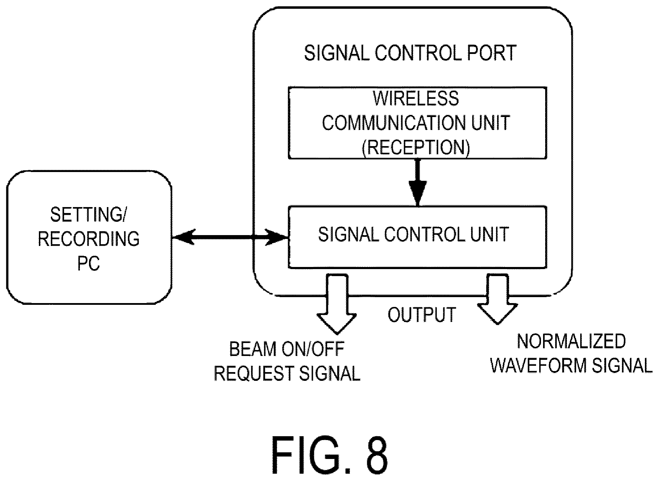

FIG. 8 is a diagram schematically illustrating a configuration of the signal control port 8 according to the present embodiment. The detection device 5 acquires a respiratory waveform immediately before each treatment. The respiratory waveform wirelessly transmitted to the signal control port 8 is normalized between the maximum exhalation position and the maximum inhalation position by a signal control unit (waveform generation unit 92) built in the signal control port 8. A setting/recording PC compares the normalized respiratory waveform with a reference respiratory waveform acquired in advance, and assigns a set level set in advance according to the comparison result. For example, the setting/recording PC assigns a set level that enables irradiation during a respiratory phase between 0% and 20%, with 0% defined as the maximum exhalation position. The setting/recording PC transmits the set level to the signal control port 8, and the signal control unit (control signal output unit 93) generates a gate signal of a rectangular wave when the respiratory phase is within the set level with respect to the respiratory waveform wirelessly received in real time.

The detection device 5 continuously acquires the respiratory waveform even during the treatment. The signal control port 8 generates a gate signal according to the set level and transmits the gate signal to the host control. When the displacement amount is detected to be within the set level, the gate signal (for example, beam request signal) is turned on, and a beam is emitted (beam ON). When the displacement amount deviated from the set level is detected, the gate signal is turned off, and the beam is immediately stopped (beam OFF).

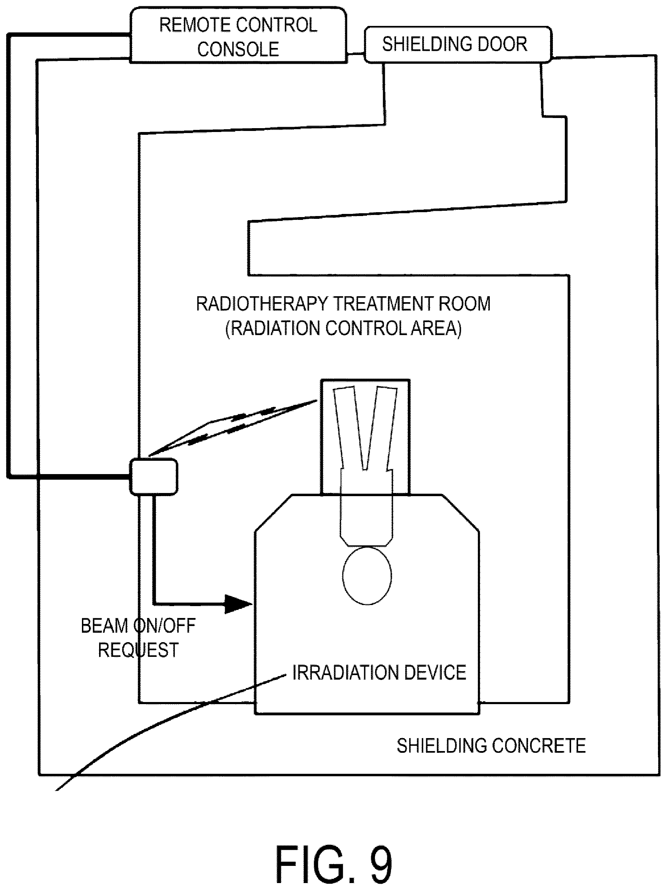

FIG. 9 is a diagram illustrating an example of use of the detection device 5 in the treatment of a respiratory moving organ according to the present embodiment. Since the radiotherapy treatment room is surrounded by shielding concrete having a thickness of about 1 to 2 m, the wireless communication is preferably limited within the treatment room, but this should not be construed in a strict sense. The signal control port 8, the setting/recording PC, and the host control may be connected by wire (for example, a wired LAN) because high-speed and stable communication is preferable. As a fail-safe, hardware such as a synchronous OFF hand switch (not illustrated) may be connected to the signal control port 8 to manually transmit a beam OFF signal to the host control.

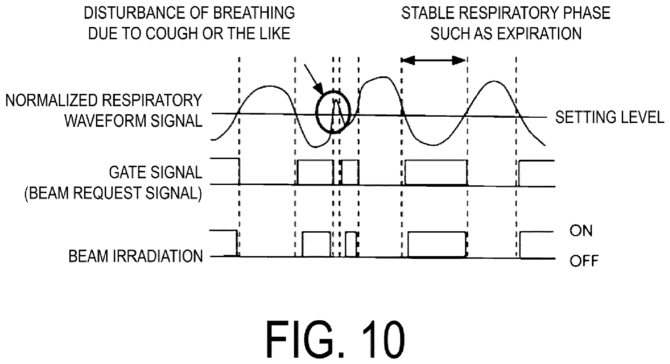

FIG. 10 is a diagram illustrating an example of a respiratory waveform received by the signal control port 8 and corresponding gate signal generation and beam irradiation control, according to the present embodiment. In the treatment system 100 according to the present embodiment, the gate signal is set only in the respiratory phase within the set level under free respiration, and the beam is emitted in accordance with the gate signal. In this case, the signal control port 8 may predict a signal from the respiratory waveform, and may perform control to turn OFF the beam before the signal exceeds the set level obtained from the actual respiratory waveform by the prediction gate signal for beam OFF in advance. When unintended disturbance of breathing occurs due to coughing or the like, control is performed so as to turn OFF the beam. Accordingly, it is possible to improve the irradiation accuracy by irradiating only a target position of an organ moving by respiration, and to minimize exposure of surrounding normal tissues.

### <Sixth Embodiment>

The irradiation of the radiotherapy itself is completed in about one minute, and the patient positioning work takes up the majority of the time required for the entire treatment session. The positioning work requires at least about 10 minutes, and, requires one hour for the respiration synchronous irradiation or the like. Thus, if such positioning work is performed in the treatment room, the use efficiency of the treatment room is compromised. Therefore, in the present embodiment, the transportable treatment table 1 (shuttle) having the function of a treatment table provided with a robot arm (driving unit 3) is used for radiotherapy, whereby the positioning work is performed in a location other than the treatment room (for example, positioning room), and the use efficiency of the treatment room is improved.

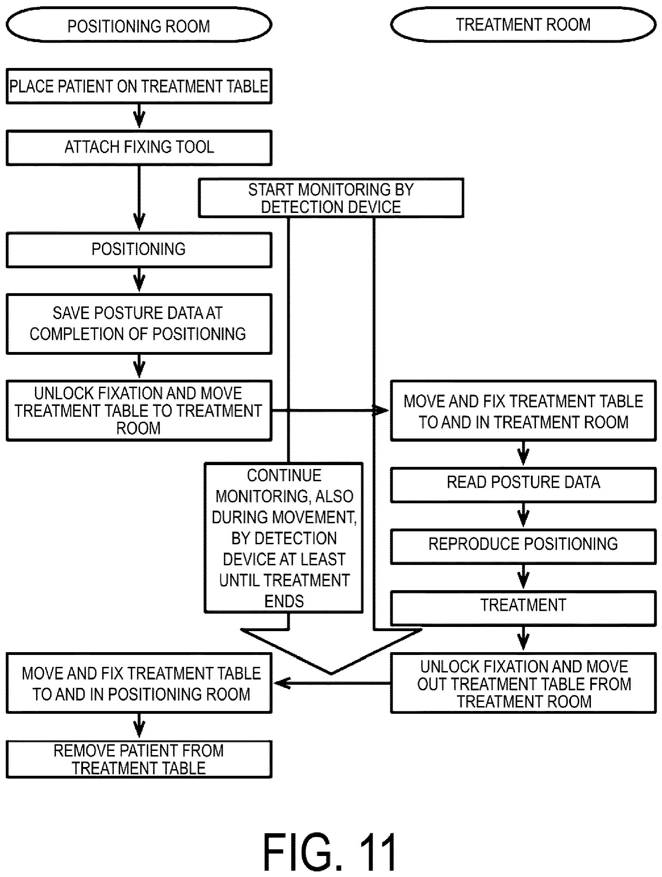

FIG. 11 is a diagram illustrating an example of a flow of treatment using the transportable treatment table 1 according to the present embodiment. In the present embodiment, after positioning of the patient placed on the placement portion 2 of the transportable treatment table 1 (laid on the treatment table and attached with the fixing tool) is performed in the positioning room, the posture data of the driving unit 3 in the positioned state (for example, six axis information (x, y, z, ψ, Φ, θ) of translation and rotation) is recorded in the host control system (for example, positioning room management device) of each room, and is transmitted to and stored in a control system (for example, the treatment control system) further on the upper level. When the fixation of the transportable treatment table 1 fixed to the positioning room is released, the patient after the positioning is transported to the treatment room while being fixed to the placement portion 2 of the transportable treatment table 1 using the fixing tool or the like. When the transportable treatment table 1 arrives at the treatment room and is fixed to the irradiation device 9 in the treatment room, the posture data stored in the treatment control system is read by the treatment room management device, and the driving unit 3 is driven based on the posture data, thereby reproducing the posture according to the positioning in the positioning room. Note that, for safety of the patient during the transportation, a posture adjusting/holding unit such as the robot arm may take a home position where the arm is stacked. When the treatment session ends, the posture adjusting/holding unit returns to the home position, and the fixation of the transportable treatment table 1 fixed in the treatment room is released, and the transportable treatment table 1 is transported to a designated stop place (which may be a positioning room or another room). The fixing tool and the like are removed, and the patient gets off the transportable treatment table 1, and the treatment ends. Here, according to the transportable treatment table 1 of the present embodiment, the monitoring by the detection device continues over a period from the start of the monitoring by the detection device with the patient placed on the placement portion 2 to at least the end of the treatment, including during the movement.

In the case of the patient of the respiration synchronous irradiation described in the present embodiment, the monitoring by the detection device 5 is preferably performed from the time of the patient positioning operation to the end of the treatment, and thus the detection device 5 is movable together with the transportable treatment table 1. In this case, with a wired detection device, time and effort are required for attachment/detachment and setting for each room, and thus the work efficiency is compromised.

Also, if the patient moves during transportation for any reason, the patient is repositioned if the change is not clinically acceptable. While a caregiver moves together with the transportable treatment table 1, it is difficult to concentrate on only the body motion of the patient. Therefore, it is preferable to use a monitor that quantitatively monitors the state of the patient during the transportation. At this time, with the detection device 5 according to the present disclosure capable of detecting a two-dimensional variable, the body motion of the patient can be detected. For example, a function of performing simple allowable value registration is incorporated in the display unit 54 (display operation unit), and in a case where the allowable value is exceeded, the alert output unit 55 of the detection device 5 outputs an alert sound, an alert display, or the like for notification to the caregiver, and the patient can quickly return to the positioning room to be repositioned.

The body motion monitoring function of the detection device 5 during the transportation on the transportable treatment table 1 is not limited to the respiration synchronous irradiation method. The body motion monitoring function is also effective for a patient who is difficult to maintain a stationary state, such as a child or an elderly person, or a patient who exhibits involuntary movement, with a disease in a head and neck or the pelvis that is a part other than the trunk portion where respiratory movement does not need to be taken into consideration for the irradiation for example. Also during the treatment session, the body motion near the irradiation site can be easily monitored at any time. In a case of a method other than respiration synchronous irradiation, conventionally, a physician, a radiologist, or the like would stops the irradiation beam manually when the patient moved in an unexpected manner (such as coughing for example). On the other hand, when the cordless scheme is employed, the detection device 5 can always monitor the displacement of any surface position. Furthermore, a beam gate signal can be transmitted, and the beam can be automatically stopped, so treatment can be performed with improved safety.

### <Seventh Embodiment>

The detection device 5 may include a plurality of units that output the voltage displacement detected by the light position detection sensor 62 to the inside and outside, such as, for example, a signal control unit, a display operation unit (display unit 54), and a wireless communication unit (communication unit 52). For example, the analog voltage displacement output from the PSD may be subjected to digital conversion and output by a sensor signal unit (not illustrated) built in the detection device 5. The output digital signal is transmitted from the communication unit 52 to the data reception unit 91 of the signal control port 8, for example. The signal control port 8 includes a signal control unit (control signal output unit 93) that outputs a normalized waveform signal and a beam ON/OFF request signal based on the received digital signal.

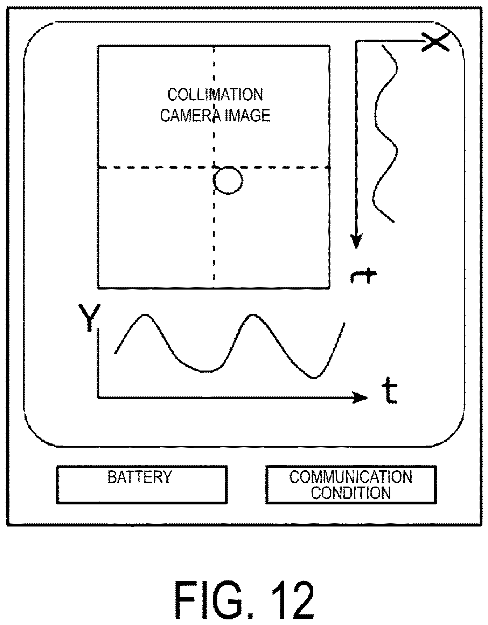

FIG. 12 is a diagram illustrating an example of information displayed on a display operation unit (display unit 54) according to the present embodiment. The digital signal described above is transmitted to the display operation unit built in the detection device 5. In the present embodiment, an image signal from the collimation camera 57 is also transmitted to the display operation unit, and the displacement amount of the living body surface may be displayed on the display unit 54 such as a liquid crystal monitor in real time as one-dimensional or two-dimensional information in synchronization with the digital signal. Since this display is a liquid crystal monitor display for positioning the reflector 56 and the main body of the detection device 5, a delay in the display is tolerated. Therefore, the display content may be a waveform after the normalization process.

### <Other Variations>

In the above-described embodiment, the signal control port 8 includes the waveform generation unit and/or the control signal output unit, and the signal control port 8 outputs the gate signal. However, instead of this configuration, the detection device 5 itself may output the gate signal. That is, the communication unit 52 may transmit control data (for example, a gate signal) corresponding to the surface position and/or the body motion displacement detected by the detection unit 51 to the external device.

### <Effects>

According to the embodiment described above, the detection device 5 has a cordless configuration, whereby space saving around the placement portion is achieved. As a result, various advantages can be obtained, such as the cable not hindering the movement of the patient or the medical staff, the cable length not being insufficient for the drive range, avoidance of interference (mechanical interference and interference with the imaging visual field or the irradiation field) with other surrounding devices (such as a medical examination device and an irradiation device), the detection device main body being installable on the patient foot side of the treatment table, not being an obstruction for a patient for which the detection device is not used, and the work required for setup being able to be shortened because the detection device main body can stay installed on the treatment table, and the safety and convenience of the treatment are improved.

Further, according to the above-described embodiment, the posture of the patient can be monitored even during the movement after the completion of the patient positioning, and when a large movement that is not clinically allowable occurs, the caregiver is alerted, and the repositioning can be immediately implemented.

According to the embodiment described above, by using a semiconductor detector such as a PSD for the light position detection sensor 62 and using high-speed wireless communication for a transmission method, the overall delay time from actual body motion to beam irradiation control is reduced as compared with the known case (for example, the overall delay time is about 10 ms or less), improvement is achieved in terms of the delay from body motion detection to beam irradiation control, and it is possible to immediately stop beam irradiation when the motion of the living body surface of the patient is not clinically acceptable.

### <Note>

Hereinafter, technical ideas that can be implemented by those skilled in the art in relation to the present disclosure will be described as notes.

### (Note 1)

A detection device comprising:
a detection unit that is installed on a treatment table including a placement portion on which a living body is placed, and configured to detect a surface position and/or a body motion displacement of the living body placed on the placement portion;
a battery that is movable together with the treatment table, and capable of supplying power to the detection unit, in a state of being disconnected from an external power supply facility;
a communication unit configured to wirelessly transmit, to an external device, data corresponding to a signal output from the detection unit; and
a connection unit configured to connect the detection unit to the treatment table, the detection unit being connected to be movable in accordance with a movement of the placement portion that is movable on the treatment table.

### (Note 2)

The detection device according to Note 1, wherein the connection unit connects the detection unit to the treatment table so that a relative positional relationship between the living body placed on the placement portion and the detection unit is maintained.

### (Note 3)

The detection device according to Note 2, wherein the connection unit maintains the relative positional relationship between the living body placed on the placement portion and the detection unit, by fixing the detection unit to the placement portion.

### (Note 4)

The detection device according to Note 1, wherein the treatment table is a transportable treatment table that is transportable to another location while the living body is placed on the placement portion.

### (Note 5)

The detection device according to Note 1, further comprising a display unit capable of displaying the surface position and/or the body motion displacement detected by the detection unit.

### (Note 6)

The detection device according to Note 1, further comprising an alert output unit configured to output an alert when the surface position and/or the body motion displacement detected by the detection unit satisfies a preset condition.

### (Note 7)

The detection device according to Note 1, wherein the communication unit transmits, to the external device, control data corresponding to the surface position and/or the body motion displacement detected by the detection unit.

### (Note 8)

The detection device according to Note 1, further comprising a reflector installed on the living body, wherein
the detection unit includes a light source and a light position detection sensor, and an optical path is configured so that incident light emitted from the light source to the living body is reflected by the reflector and reflected light from the reflector is input to the light position detection sensor.

### (Note 9)

The detection device according to Note 8, further comprising a camera capable of capturing an image in a direction substantially same as a direction in which the reflected light from the reflector is obtained.

### (Note 10)

The detection device according to Note 8, wherein the detection unit further includes one or a plurality of optical members configured to control a trajectory of the incident light and/or the reflected light.

### (Note 11)

The detection device according to Note 10, wherein
the one or plurality of optical members include at least one of:
a half mirror configured to substantially align an optical axis of the incident light and an optical axis of the reflected light in a section between the reflector and the one or plurality of optical members and separate the optical axes in a section between the one or plurality of optical members and the light source or the light position detection sensor; and
a reflection mirror that is provided in a section between the reflector and the light position detection sensor to control the optical axis of the incident light and the optical axis of the reflected light in the section.

### (Note 12)

The detection device according to Note 10, further comprising a camera capable of capturing an image in a direction in which the reflected light from the reflector is obtained, wherein
at least one optical member of the one or plurality of optical members is a splitter configured to separate input light from the living body side including the reflected light into light including at least the reflected light entering the light position detection sensor and light including at least visible light from the living body side entering the camera.

### (Note 13)

The detection device according to Note 8, wherein the reflector is a retroreflector.

### (Note 14)

A transportable treatment table that is transportable to another location while a living body is placed thereon, the transportable treatment table comprising:
a placement portion on which a living body is placed;
a detection unit configured to detect a surface position and/or a body motion displacement of the living body placed on the placement portion;
a battery that is movable together with the transportable treatment table, and capable of supplying power to the detection unit, in a state of being disconnected from an external power supply facility;
a communication unit configured to wirelessly transmit, to an external device, data corresponding to a signal output from the detection unit;
a connection unit configured to connect the detection unit to the transportable treatment table, the detection unit being connected to be movable in accordance with a movement of the placement portion that is movable on the transportable treatment table; and
a transport unit making the transportable treatment table to be movable.

### (Note 15)

The transportable treatment table according to Note 14, wherein the battery supplies power to the transport unit in addition to the detection unit.

### (Note 16)

The transportable treatment table according to Note 14, further comprising a driving unit configured to translate and/or rotate the placement portion, wherein
the battery supplies power to the driving unit in addition to the detection unit.

### (Note 17)

A treatment system comprising:
a transportable treatment table that is transportable to another location while a living body is placed thereon;
an irradiation device configured to perform beam irradiation on the living body for treatment; and
a signal control device configured to control the irradiation device in accordance with data input, wherein
the transportable treatment table includes:
   a placement portion on which a living body is placed;
   a detection unit configured to detect a surface position and/or a body motion displacement of the living body placed on the placement portion;
   a battery that is movable together with the transportable treatment table, and capable of supplying power to the detection unit, in a state of being disconnected from an external power supply facility;
   a communication unit configured to wirelessly transmit, to an external device, data corresponding to a signal output from the detection unit;
   a connection unit configured to connect the detection unit to the transportable treatment table, the detection unit being connected to be movable in accordance with a movement of the placement portion that is movable on the transportable treatment table; and
   a transport unit making the transportable treatment table to be movable, and
   the signal control device includes:
      a data reception unit configured to receive the data wirelessly transmitted from the communication unit; and
      a control signal output unit configured to output a signal for enabling control of the beam irradiation by the irradiation device, based on the data received.

### (Note 18)

The treatment system according to Note 17, wherein
the signal control device further includes a waveform generation unit configured to generate a waveform representing the surface position and/or the body motion displacement of the living body based on the data received, and
the control signal output unit outputs, to the irradiation device, the signal for controlling the beam irradiation so as to perform the beam irradiation at a timing satisfying a predetermined condition for determining that the surface position and/or the body motion displacement in the waveform is stable.

### (Note 19)

The detection device comprising:
a detection unit that is installed on a treatment table including a placement portion on which a living body is placed, and configured to detect a surface position and/or body motion displacement of the living body placed on the placement portion; and
a reflector installed on the living body, wherein
the detection unit includes a light source and a light position detection sensor, and an optical path is configured so that incident light emitted from the light source to the living body is reflected by the reflector and reflected light from the reflector is input to the light position detection sensor.

### (Note 20)

The detection device according to Note 19, further comprising a camera capable of capturing an image in a direction substantially same as a direction in which the reflected light from the reflector is obtained.

### (Note 21)

The detection device according to Note 19, wherein the detection unit further includes one or a plurality of optical members configured to control a trajectory of the incident light and/or the reflected light.

### (Note 22)

The detection device according to Note 21, wherein
the one or plurality of optical members include at least one of:
a half mirror configured to substantially align an optical axis of the incident light and an optical axis of the reflected light in a section between the reflector and the one or plurality of optical members and separate the optical axes in a section between the one or plurality of optical members and the light source or the light position detection sensor; and
a reflection mirror that is provided in a section between the reflector and the light position detection sensor to control the optical axis of the incident light and the optical axis of the reflected light in the section.

### (Note 23)

The detection device according to Note 21, further comprising a camera capable of capturing an image in a direction in which the reflected light from the reflector is obtained, wherein
at least one optical member of the one or plurality of optical members is a splitter configured to separate input light from the living body side including the reflected light into light including at least the reflected light entering the light position detection sensor and light including at least visible light from the living body side entering the camera.

### Reference Signs List

1 Transportable treatment table
5 Detection device

## Claims

1. A detection device comprising:
a detection unit that is installed on a treatment table including a placement portion on which a living body is placed, and configured to detect a surface position and/or a body motion displacement of the living body placed on the placement portion;
a battery that is movable together with the treatment table, and capable of supplying power to the detection unit, in a state of being disconnected from an external power supply facility;
a communication unit configured to wirelessly transmit, to an external device, data corresponding to a signal output from the detection unit; and
a connection unit configured to connect the detection unit to the treatment table, the detection unit being connected to be movable in accordance with a movement of the placement portion that is movable on the treatment table.

2. The detection device according to claim 1, wherein the connection unit connects the detection unit to the treatment table so that a relative positional relationship between the living body placed on the placement portion and the detection unit is maintained.

3. The detection device according to claim 2, wherein the connection unit maintains the relative positional relationship between the living body placed on the placement portion and the detection unit, by fixing the detection unit to the placement portion.

4. The detection device according to claim 1, wherein the treatment table is a transportable treatment table that is transportable to another location while the living body is placed on the placement portion.

5. The detection device according to claim 1, further comprising a display unit capable of displaying the surface position and/or the body motion displacement detected by the detection unit.

6. The detection device according to claim 1, further comprising an alert output unit configured to output an alert when the surface position and/or the body motion displacement detected by the detection unit satisfies a preset condition.

7. The detection device according to claim 1, wherein the communication unit transmits, to the external device, control data corresponding to the surface position and/or the body motion displacement detected by the detection unit.

8. The detection device according to claim 1, further comprising a reflector installed on the living body, wherein
the detection unit includes a light source and a light position detection sensor, and an optical path is configured so that incident light emitted from the light source to the living body is reflected by the reflector and reflected light from the reflector is input to the light position detection sensor.

9. The detection device according to claim 8, further comprising a camera capable of capturing an image in a direction substantially same as a direction in which the reflected light from the reflector is obtained.

10. The detection device according to claim 8, wherein the detection unit further includes one or a plurality of optical members configured to control a trajectory of the incident light and/or the reflected light.

11. The detection device according to claim 10, wherein
the one or plurality of optical members include at least one of:
a half mirror configured to substantially align an optical axis of the incident light and an optical axis of the reflected light in a section between the reflector and the one or plurality of optical members and separate the optical axes in a section between the one or plurality of optical members and the light source or the light position detection sensor; and
a reflection mirror that is provided in a section between the reflector and the light position detection sensor to control the optical axis of the incident light and the optical axis of the reflected light in the section.

12. The detection device according to claim 10, further comprising a camera capable of capturing an image in a direction in which the reflected light from the reflector is obtained, wherein
at least one optical member of the one or plurality of optical members is a splitter configured to separate input light from the living body side including the reflected light into light that includes at least the reflected light to enter the light position detection sensor and light that includes at least visible light from the living body side to enter the camera.

13. The detection device according to claim 8, wherein the reflector is a retroreflector.

14. A transportable treatment table that is transportable to another location while a living body is placed thereon, the transportable treatment table comprising:
a placement portion on which a living body is placed;
a detection unit configured to detect a surface position and/or a body motion displacement of the living body placed on the placement portion;
a battery that is movable together with the transportable treatment table, and capable of supplying power to the detection unit, in a state of being disconnected from an external power supply facility;
a communication unit configured to wirelessly transmit, to an external device, data corresponding to a signal output from the detection unit;
a connection unit configured to connect the detection unit to the transportable treatment table, the detection unit being connected to be movable in accordance with a movement of the placement portion that is movable on the transportable treatment table; and
a transport unit making the transportable treatment table to be movable.

15. The transportable treatment table according to claim 14, wherein the battery supplies power to the transport unit in addition to the detection unit.

16. The transportable treatment table according to claim 14, further comprising a driving unit configured to translate and/or rotate the placement portion, wherein
the battery supplies power to the driving unit in addition to the detection unit.

17. A treatment system comprising:
a transportable treatment table that is transportable to another location while a living body is placed thereon;
an irradiation device configured to perform beam irradiation on the living body for treatment; and
a signal control device configured to control the irradiation device in accordance with data input, wherein
the transportable treatment table includes:
a placement portion on which a living body is placed;
a detection unit configured to detect a surface position and/or a body motion displacement of the living body placed on the placement portion;
a battery that is movable together with the transportable treatment table, and capable of supplying power to the detection unit, in a state of being disconnected from an external power supply facility;
a communication unit configured to wirelessly transmit, to an external device, data corresponding to a signal output from the detection unit;
a connection unit configured to connect the detection unit to the transportable treatment table, the detection unit being connected to be movable in accordance with a movement of the placement portion that is movable on the transportable treatment table; and
a transport unit making the transportable treatment table to be movable, and
the signal control device includes:
a data reception unit configured to receive the data wirelessly transmitted from the communication unit; and
a control signal output unit configured to output a signal for enabling control of the beam irradiation by the irradiation device, based on the data received.

18. The treatment system according to claim 17, wherein
the signal control device further includes a waveform generation unit configured to generate a waveform representing the surface position and/or the body motion displacement of the living body, based on the data received, and
the control signal output unit outputs, to the irradiation device, the signal for controlling the beam irradiation so as to perform the beam irradiation at a timing satisfying a predetermined condition for determining that the surface position and/or the body motion displacement in the waveform is stable.
